# DEMANDE DE BREVET EUROPEEN

(11) **EP 2 606 876 A2**
(43) Date de publication de la demande: **26.06.2013**
(21) Numéro de dépôt: 12197583.3
(22) Date de dépôt: 17.12.2012
(51) Int. Cl.: A61K 8/85, A61Q 1/10

(54) **Composition de maquillage de fibres kératiniques**

(30) Priorité: 16.12.2011 LU 91919
(71) Demandeur: Luxcos S.A., 3225 Bettembourg (LU)
(72) Inventeur: Thiel, Corinne, 57100 Thionville (FR); Delas, Christophe, 3926 Mondercange (LU)
(74) Mandataire: Office Freylinger

(57) **Abrégé**

L'invention décrit une composition de maquillage pour fibres, en particulier de fibres kératiniques, comprenant au moins une phase lipidique, au moins un pigment, au moins un agent filmogène, au moins une phase aqueuse et une quantité efficace d'au moins un agent d'amélioration de l'intensité de la couleur, le ou les agent(s) d'amélioration de l'intensité de la couleur étant un copolymère par condensation d'acide adipique et de pentaérythritol comprenant des groupes terminaux d'acide caproïque, d'acide heptanoïque, d'acide caprylique et/ou d'acide caprique ou un mélange de plusieurs de ces copolymères. De plus, l'invention concerne l'utilisation desdits copolymères comme agent d'amélioration de l'intensité de la couleur dans une telle composition, ainsi qu'un procédé de préparation d'une telle composition de maquillage.

## Description

### Domaine technique

La présente invention concerne d'une manière générale une composition de maquillage des fibres kératiniques.

### Etat de la technique

Les différentes propriétés que l'on demande à une composition de maquillage des fibres kératiniques et autres (mascara notamment) sont :
- Une bonne application à l'aide d'une brosse type goupillon constituée de fibres de nylon maintenues par un fil torsadé ou à l'aide d'une brosse dite « élastomère ».
- Un effet immédiat. Les consommatrices apprécient généralement les formules ou plutôt les combinaisons formules/packaging qui permettent d'obtenir l'effet recherché après quelques applications seulement.
- Un temps de séchage approprié en fonction de l'effet recherché. Les formules pour lesquelles l'effet « longlash » ou l'effet « curl » sont recherchés auront généralement un temps de séchage plus court que les formules pour lesquelles un effet volume est souhaité.
- Une tenue de la formule dans le temps. La formule ne doit pas s'effriter dans le temps ou former des petites particules qui tombent sur la joue (phénomène appelé « flaking »). Et la formule ne doit pas transférer sur la paupière sous la forme de traces noires (phénomène appelé « smudge »).
- Une tenue de l'effet dans le temps. Il est en effet important que l'effet obtenu lors de l'application (effet volume, courbé, séparation des cils, ...) se conserve au cours de la journée.

Le paramètre application de la composition sur les cils dépend de nombreux paramètres physico-chimiques en particulier la viscosité, la nature des cires et de leur quantité utilisée, ainsi que la quantité des charges mises en oeuvre. Dans la pratique, elle dépend également fortement de l'applicateur mis en jeu et de la dureté/rigidité des fibres utilisées pour fabriquer cet applicateur.

L'effet apporté par la formule est en relation directe avec les ingrédients utilisés pour préparer la composition et l'applicateur utilisé. Le développement d'un produit (combinaison formule/packaging) implique généralement l'utilisation d'un applicateur défini en fonction de l'effet ou des performances recherchés.

Le temps de séchage est fortement dépendant de trois paramètres :
- Le taux de matière sèche de la formule. Plus le taux de matière sèche est élevé, plus le temps de séchage sera long.
- La viscosité de la formule.
- La quantité appliquée sur les fibres kératiniques ou (faux-)cils.

La tenue dans le temps est en relation directe avec les ingrédients mis en jeu dans la formule. Pour éviter les phénomènes de « flaking » par exemple, les cires trop dures ainsi que les charges en quantité trop élevées seront évitées. Les phénomènes de « smudge » seront évités en réalisant des formules dont la phase lipidique n'est pas prédominante par rapport à la phase aqueuse. Le « smudge » est généralement un phénomène de transfert de la formule sèche des cils vers la paupière ou la zone située directement sous les yeux.

Bien que la coloration de la composition provienne en principe du ou des pigments utilisés, la couleur effective de la composition dépend fortement de la nature des autres ingrédients utilisés au sein de la formulation, en particulier les cires et les dispersions de polymère éventuellement mises en oeuvre, elle peut être terne et peut même paraître « grisâtre » dans le cas de l'utilisation d'oxyde de fer noir dans la composition par exemple.

De plus, au vu de la grande variété d'ingrédients utilisés et utilisables dans ce genre de compositions, il existe un réel besoin de mieux maîtriser les résultats obtenus, en particulier bien sûr en ce qui concerne le rendu de couleur ou l'intensité de la couleur. En effet, pour la consommatrice cette caractéristique est un critère important puisque le rendu de couleur affecte directement l'appréciation esthétique générale de la composition.

### Objet de l'invention

Un objet de la présente invention est par conséquent de proposer une formulation de compositions cosmétiques ou de maquillage applicables sur les fibres kératiniques, en particulier les cils humains (ou même les faux-cils), qui permettent un rendu de couleur plus intense.

Conformément à l'invention, cet objectif est atteint par une composition selon la revendication 1, respectivement une utilisation selon la revendication 12.

### Description générale de l'invention

Afin de résoudre le problème mentionné ci-dessus, la présente invention propose une composition de maquillage pour fibres, en particulier de fibres kératiniques, comprenant au moins une phase lipidique, au moins un pigment, au moins un agent filmogène, au moins une phase aqueuse et une quantité efficace d'au moins un agent d'amélioration de l'intensité de la couleur. Selon l'invention, le ou les agent(s) d'amélioration de l'intensité de la couleur est/sont un copolymère obtenu par condensation d'acide adipique et de pentaérythritol comprenant des groupes terminaux d'acide caproïque, d'acide heptanoïque, d'acide caprylique et/ou d'acide caprique ou un mélange de plusieurs de ces copolymères.

En effet, les inventeurs ont découvert d'une manière surprenante que lesdits copolymères par condensation d'acide adipique et de pentaérythritol permettent d'améliorer la dispersion et le mouillage des pigments organiques et/ou inorganiques mis en oeuvre. Cette amélioration se caractérise en particulier par une couleur plus intense du mascara sur la brosse mais aussi sur les cils. De plus, cet effet est non seulement immédiat, c'est-à-dire directement obtenu à l'application, mais il est également significativement plus durable dans le temps.

Les copolymères utilisables sont ceux pouvant être obtenus par condensation d'acide adipique et de pentaérythritol d'une part et par addition de groupes terminaux choisis parmi l'acide caproïque, l'acide heptanoïque, l'acide caprylique, l'acide caprique, ou l'une quelconque des combinaisons de ces acides d'autre part. En général, ces copolymères possèdent une viscosité à 25°C de l'ordre de 700 mPa.s, par exemple de 600 à 800 mPa.s, de manière préférée entre 650 et 750 mPa.s. De préférence, le copolymère est un copolymère d'acide adipique et de pentaérythritol à groupes terminaux d'acide caproïque, d'acide heptanoïque, d'acide caprylique et d'acide caprique, c'est-à-dire le polyester-4 (désignation INCI).

Bien que le polyester-4 ait déjà été proposé dans le cadre de compositions « cosmétiques », comme par exemple dans le document EP 1 240 894 A2 ou dans le document EP 2 353 578 A2, aucun de ces documents ne concerne des compositions de maquillage pour fibres, en particulier de fibres kératiniques, autrement dit des « mascaras ». En effet, le premier document concerne d'une manière générale des compositions de protection solaire et plus spécifiquement des compositions pour les lèvres. Accessoirement, ce document cite parmi d'autres exemples, des compositions comprenant du polyester-4. Le deuxième document concerne également des compositions pour les lèvres, mais comprenant des particules galvaniques. Tout comme le premier document, ce document ne cite qu'accessoirement, parmi de nombreux autres ingrédients, le polyester-4. Or, ni l'un, ni l'autre ne mentionnent un quelconque effet particulier de ce composé et surtout pas ses propriétés surprenantes d'amélioration de l'intensité des couleurs mises en évidence par la présente invention.

Par l'expression « quantité efficace d'au moins un agent d'amélioration de l'intensité de la couleur », on entend une quantité suffisante pour obtenir une amélioration notable et significative du rendu de couleur (au plus tard) après application de la composition. Cette quantité minimale en agent d'amélioration du rendu de couleur ou de la brillance à mettre en oeuvre, qui peut varier selon la nature du type de composition retenue (émulsion huile/eau, émulsion eau/huile), ainsi qu'en fonction des autres ingrédients choisis, peut être déterminée sans aucune difficulté par l'application du mascara sur une plaque de verre à l'aide d'un applicateur 100 µm. Le mascara est, avant et après séchage, visiblement plus noir par rapport à la formule sans agent d'amélioration du rendu de couleur. En pratique, le ou les agent(s) d'amélioration de l'intensité de la couleur représente(nt) en général 0.01 à 30 % en poids, de préférence 0.1 à 20 % en poids, en particulier de 0.2 à 10 % en poids de la composition totale.

Comme déjà mentionné, le premier avantage de la présente invention consistant à proposer l'utilisation d'un ou de plusieurs copolymères obtenus par condensation d'acide adipique et de pentaérythritol comprenant des groupes terminaux d'acide caproïque, d'acide heptanoïque, d'acide caprylique et/ou d'acide caprique est l'accroissement significatif de l'intensité des couleurs de la formulation dès l'application, c'est-à-dire que, contrairement à d'autres agents connus, l'intensité des couleurs est directement améliorée. Le deuxième avantage est que l'effet d'intensification de la couleur est plus durable que pour de nombreux agents connus. Il en résulte le double effet d'un taux d'intensité plus important au départ, combiné à une constance élevée de l'intensité pendant plusieurs heures après application.

D'autre part, l'ajout des agents d'amélioration de l'intensité de la couleur selon l'invention n'affecte pas négativement les autres qualités des compositions, à savoir les bonnes caractéristiques d'application et le temps de séchage.

Un avantage additionnel important de la présente invention est que les compositions de maquillage pour fibres à rendu de couleur amélioré peuvent comprendre les ingrédients habituellement utilisés par l'homme de l'art. L'homme de métier n'est donc pas obligé de reconsidérer tous les ingrédients et leurs effets, s'il souhaite utiliser les présents agents d'amélioration de l'intensité de la couleur. Les présents agents d'amélioration de l'intensité de la couleur présentent par conséquent une très bonne compatibilité avec les ingrédients courants dans le domaine.

Les formulations selon l'invention se présentent en pratique généralement sous la forme d'émulsions. Une émulsion est un mélange stable de deux liquides non miscibles. Deux types d'émulsions sont envisageables : les émulsions huile dans l'eau (H/E) et les émulsions eau dans l'huile (E/H). Les émulsions H/E forment généralement des mascaras peu résistants à l'eau sous réserve de l'addition de polymères spécifiques. Les émulsions E/H forment des mascaras ayant une haute résistante à l'eau et sont généralement décrites comme des mascaras « waterproof » par l'homme de l'art.

Dans une variante, la composition de maquillage pour fibres comprend en plus au moins une phase aqueuse et représente une émulsion de type huile dans eau (H/E) comprenant entre 30% et 80% en poids, de préférence entre 40% et 70% en poids d'eau par rapport à la composition totale. Dans ces formulations, l'eau est donc l'un des constituants majoritaires.

Dans une autre variante, la composition de maquillage pour fibres comprend également en plus au moins une phase aqueuse, mais représente une émulsion de type eau dans huile (E/H) qui comprend entre 50% et 95% en poids, de préférence entre 60% et 90% en poids de composant lipidique par rapport à la composition totale.

Ces compositions peuvent contenir le cas échéant les ingrédients ou types d'ingrédients suivants :

Des solvants ou cosolvants. Ces cosolvants permettent de solubiliser des ingrédients qui ont une solubilité faible dans l'eau ou encore d'accélérer dans certains cas le temps de séchage de la composition. Parmi ces solvants miscibles à l'eau, on citera l'éthanol, l'isopropanol, les glycols comme le propylène glycol, l'éthylène glycol, le 1,2-butylène glycol ou le dipropylène glycol.

Des cires. On considérera les cires comme des corps gras solides à température ambiante (25°C), et qui seront miscibles dans des huiles en les portant à l'état liquide. Les cires peuvent être d'origines variées :
- Les cires d'origine animale comme la cire d'abeille ainsi que les cires d'abeille modifiées ou la cire de lanoline.
- Les cires d'origine végétale comme la cire de carnauba, la cire de candellila, la cire de son de riz, la cire de berry, la cire d'alfa, la cire de shellac, la cire du Japon, la cire d'orange, la cire de citron.
- Les cires d'origine minérale comme la cire de paraffine, la cire microcristalline, l'ozokérite, la cérésine.
- Des cires d'origines synthétiques comme les cires de polyéthylène, les cires obtenues par la synthèse de Fisher-Tropsch, les cires de silicone, les cires fluorées.

D'une manière générale, une composition selon l'invention peut contenir une teneur en cire allant jusqu'à 40% en poids, généralement entre 10% et 35% et de préférence entre 15% et 30% en poids de la composition totale.

Des corps gras cireux :
- Les alcools gras comme l'alcool cétylique, l'alcool stéarique, l'alcool oléique
- Les dérivés de l'acide stéarique comme le stéarate de glycéryle, les stéarates de polyéthylène glycol,
- Les dérivés de rosine comme le rosinate hydrogéné de glycéryle, le rosinate hydrogéné de méthyle
- Les esters d'acide gras comme l'ester de la glycérine et d'acides gras en C18-36, les esters d'olive hydrogénés.
- Les beurres. On citera par exemple le beurre de karité.

Les corps gras cireux peuvent être présents dans la composition à des teneurs comprises ente 0.1% et 15% et de préférence à des teneurs comprises entre 0.5% et 10% en poids de la composition totale.

Des huiles volatiles ou non volatiles peuvent être contenues dans des proportions comprises entre 0.1% et 15% et de préférence entre 0.1% et 7% en poids de la composition totale:
- Huiles volatiles comme les diméthicones ou les cyclométhicones, l'isododécane, l'isohexadécane ou l'isodécane.
- Huiles non volatiles comme l'huile de paraffine, l'huile de ricin, l'huile de ricin hydrogénée, les dérivés estérifiés d'olive, le polydécène hydrogéné, le propylène glycol, comme les esters de Guerbet (isononanoate d'isotridécyle, neopentanoate d'isostéaryle, néopentanoate de tridécyle, octanoate de cétyle), les huiles silicones non volatiles (amodiméthicone, phényltriméthicone, phényltrimethicone).

Des filmogènes. Parmi ces filmogènes, on peut citer :
- Les polymères d'ester vinylique comme par exemple le polyvinylacétate, ou encore les copolymères d'esters vinyliques.
- Les copolymères de polyvinylpyrrolidone tels que les copolymères vinylpyrrolidone et triacontane, les copolymères vinylpyrrolidone et éicosène, et les copolymères vinylpyrrolidone et hexadécène.
- Les polymères de cellulose et ses dérivés : sodium hydroxyéthylcellulose, carboxyméthylcellulose, cétyl hydroxyéthylcellulose, hydroxyéthylcellulose. Ces polymères sont commercialisés selon plusieurs grades qui se caractérisent notamment par des longueurs de chaîne différentes.
- Les ingrédients décrits sous la dénomination polyquaternium. Ce sont des polymères cationiques qui ont des actions filmogènes mais peuvent aussi jouer le rôle d'agent antistatique.
- Les copolymères éthylène / propylène / styrène ou les copolymères butylène /éthylène / styrène contenus par exemple dans les ingrédients vendus sous la dénomination Versagel.
- Les polyesters obtenus par polycondensation d'acides dicarboxyliques avec des polyols en particulier des diols. Les diols et les acides dicarboxyliques peuvent être aromatiques, aliphatiques ou alicycliques.
- Les résines siloxysilicates comme le triméthylsiloxysilicate ou les copolymères de résines de silicone.
- Les polymères acryliques qui se présentent généralement sous la forme de dispersions aqueuses ou d'émulsions. Parmi ces polymères on citera les copolymères et homopolymères acryliques et les copolymères styrène /acryliques.
- Les polyuréthanes qui se différencient notamment par la nature des monomères qui les constituent. Ce type de polymère se présente également sous la forme d'émulsions ou de dispersions aqueuses.

Ces filmogènes (ou leurs mélanges) peuvent être utilisés entre 0.5% et 25% en poids de la composition et de préférence entre 1% et 15% en poids de la formulation.

Des pigments. Les pigments peuvent être colorés ou blancs, organiques et/ou minéraux. Ces pigments sont susceptibles d'avoir subi un traitement particulier ou non. La teneur en pigment(s) se situe en générale entre 0.1 et 15% en poids de la composition totale. Parmi ces pigments on peut citer notamment :
- Les pigments inorganiques :
   o Les oxydes de fer noirs, bruns, rouges et jaunes. Ces oxydes de fer se différencient notamment par leur degré d'oxydation ainsi que leur niveau d'hydratation.
   o Les oxydes de chrome et les hydrates de chrome qui permettent d'obtenir des nuances variables de verts.
   o Le bleu outremer.
   o Le bleu ferrique ferrocyanide.
   o Le dioxyde de titane.
   o Le violet de manganèse.
- Les pigments organiques :
   o Le noir de carbone.
   o Les pigments de type D&C à base de baryum, strontium, calcium et aluminium ou encore le pigment obtenu à partir de la cochenille (ce pigment est également connu sous le nom de carmin).
- Les nacres qui peuvent être constituées de mica naturel ou synthétique (fluorphlogopite synthétique), par exemple recouvert de dioxyde de titane, d'oxyde de fer, ou toute autre matière colorante de nature organique ou inorganique.

Des charges. Les charges peuvent être minérales ou organiques, lamellaires ou sphériques. Les charges utilisables sont incolores ou blanches (non pigmentaires). Parmi les charges utiles, on peut citer :
- Les charges minérales telle que le talc, le mica, la silice ou le kaolin.
- Les charges synthétiques comme les poudres de polyamide (Nylon®), comme les poudres de polymères de tétrafluoroéthylène (Téflon®), comme les particules de polyméthacrylate de méthyle, les poudres de polyuréthane.
- Les charges organiques comme la lauroyl-lysine, l'amidon et ses dérivés.

Les charges (ou une combinaison de plusieurs de celles-ci) sont généralement présentes en une teneur d'au moins 0.1% en poids par rapport à la composition totale et de préférence entre 0.1 % et 10% en poids de la composition totale.

Des fibres. Ces fibres peuvent notamment permettre d'améliorer l'effet d'allongement de la formule. Les fibres ont généralement une longueur pouvant aller de 0.01 mm à 10 mm et en particulier de 0.1 à 5 mm. Les fibres utilisables sont par exemple les fibres de polyamides (Nylon®).

Des agents gélifiants comme les homo- ou copolymères d'acide acrylique ou d'acide méthacrylique, les acides polyacryliques, le polyméthacrylate de sodium, les polymères de cellulose, les gommes arabiques, les gommes de xanthane. Ces différents gélifiants peuvent être utilisés à des teneurs comprises entre 0.1% et 10%en poids de la composition et de préférence entre 0.1% et 5% de la composition.

Des Conservateurs. Ces ingrédients ont pour fonction de stabiliser la formule d'un point de vue bactériologique. Les conservateurs susceptibles de faire partie de ce type de formulation sont : le phénoxyéthanol, le propylparabène, le méthylparabène, le butylparabène, l'éthylparabène, l'isobutylparabène, le disodium EDTA, le tétrasodium EDTA, le déshydroacétate de sodium, le sorbate de potassium, l'acide benzoïque, le benzoate de sodium, l'imidazolidinylurée, l'alcool benzylique, la chlorophénésine. Si ces ingrédients sont utilisés au sein de la composition, les teneurs suivront les prescriptions des réglementations cosmétiques en vigueur. Elles seront utilisées dans un pourcentage minimum, à condition que ce pourcentage garantisse l'intégrité bactériologique de la composition dans des conditions normales d'utilisation.

Des antioxydants comme le BHT, le TBHQ, la vitamine E dans des teneurs comprises en général entre 0.05% et 0.3% en poids de la composition totale.

Des résines d'origine végétale comme la colophane, la shellac ou encore la résine élémi.

Des agents tensioactifs émulsionnants et des co-émulsionnants. Ces émulsionnants sont choisis de manière appropriée pour l'obtention de l'émulsion désirée. Ces agents tensioactifs peuvent être choisis parmi des agents non ioniques, anioniques, cationiques ou amphotères. Plusieurs types d'émulsionnants peuvent être envisagés pour la réalisation de ce type d'émulsion :
- Les « savons » de l'acide stéarique ou de l'acide palmitique. Ces acides gras peuvent être neutralisés par la triéthanolamine, l'aminométhylpropanol, l'aminométhylpropanediol, l'hydroxide de potassium ou encore l'hydroxyde de sodium.
- Les dérivés de phosphate comme le cétyl phosphate ou le cétyl phosphate de potassium.

Des actifs cosmétiques peuvent être utilisés dans la composition selon l'invention. Parmi ces actifs, on citera notamment les parfums, les émollients, les vitamines et les filtres solaires.

Les compositions selon l'invention sont de préférence conditionnées dans un récipient hermétique. Ce même récipient comprend généralement un applicateur se présentant sous la forme d'une brosse comportant des poils maintenus par un fil torsadé ou sous la forme d'une brosse élastomère ou encore sous la forme d'un peigne, obtenus par moulage. Ces applicateurs sont généralement associés à une tige portée par l'élément de fermeture.

Les compositions applicables sur les fibres kératiniques comme les mascaras ont généralement un aspect pâteux ou crémeux dont la couleur dépend du pourcentage et de la nature du ou des matières colorantes mises en oeuvre.

La consistance de la formule est également dépendante des teneurs et de la nature des agents gélifiants, des charges, des cires ou encore des tensioactifs émulsionnants utilisés.

Dans un aspect supplémentaire, l'invention propose donc l'utilisation d'un un ou de plusieurs copolymères obtenus par condensation d'acide adipique et de pentaérythritol comprenant des groupes terminaux d'acide caproïque, d'acide heptanoïque, d'acide caprylique et/ou d'acide caprique comme agent(s) d'amélioration de l'intensité de la couleur dans la préparation de compositions de maquillage pour fibres, en particulier de fibres kératiniques. De préférence, la quantité de copolymère(s) par condensation d'acide adipique et de pentaérythritol comprenant des groupes terminaux d'acide caproïque, d'acide heptanoïque, d'acide caprylique et/ou d'acide caprique représente de 0.01 à 30 % en poids, de préférence de 0.1 à 20 % en poids, en particulier de 0.2 à 10 % en poids de la composition cosmétique totale.

L'invention concerne également un procédé de préparation d'une telle composition de maquillage pour fibres, en particulier de fibres kératiniques, notamment un mascara, comprenant le mélange d'au moins une phase lipidique, d'au moins un pigment, d'au moins un agent filmogène et d'au moins un agent d'amélioration de l'intensité de la couleur, le ou les agent(s) d'amélioration de l'intensité de la couleur étant choisi(s) parmi les copolymères par condensation d'acide adipique et de pentaérythritol comprenant des groupes terminaux d'acide caproïque, d'acide heptanoïque, d'acide caprylique et/ou d'acide caprique ou leurs mélanges, ainsi qu'éventuellement au moins une phase aqueuse, et/ou éventuellement un ou plusieurs autres additifs choisis parmi les charges, les fibres, les agents gélifiants, les agents conservateurs, les antioxydants, les résines d'origine végétale, les agents tensioactifs émulsionnants et des co-émulsionnants, les parfums, les émollients, les vitamines et les filtres solaires.

Comme avantage supplémentaire de l'invention, en complément des avantages déjà mentionnés ci-dessus en relation avec les compositions, il est à remarquer que le procédé ci-dessus peut être réalisé moyennant des installations tout à fait standard et ne demande pas de connaissances, ni de précautions particulières de l'opérateur.

Un autre avantage majeur de l'invention réside dans le fait que, grâce à l'utilisation des agents d'amélioration de l'intensité de la couleur à base d'un ou de plusieurs copolymères par condensation d'acide adipique et de pentaérythritol comprenant des groupes terminaux d'acide caproïque, d'acide heptanoïque, d'acide caprylique et/ou d'acide caprique, la fabrication de telles compositions ne nécessite pas d'étape de broyage mécanique des agents colorants utilisés lors de la fabrication et en particulier des pigments organiques ou inorganiques.

Il est en effet connu de l'homme de l'art que l'utilisation de pigments organiques ou inorganiques dans certains types de formulations cosmétiques nécessite généralement une étape de broyage qui a pour but de :
- Casser les agglomérats de pigments; les agglomérats correspondent à l'association de structures primaires de pigments par des contacts de surface.
- Casser les agrégats de pigments; les agrégats correspondent plutôt à une structure secondaire ayant pour origine l'association des structures primaires par des contacts bord à bord.

En conclusion, les inventeurs ont découvert d'une manière surprenante que lesdits copolymères par condensation d'acide adipique et de pentaérythritol comprenant des groupes terminaux d'acide caproïque, d'acide heptanoïque, d'acide caprylique et/ou d'acide caprique ou leurs mélanges, permettaient de réaliser l'objectif d'améliorer la couleur d'un mascara ou d'une composition de ce type.

Cette amélioration se caractérise notamment par une couleur plus intense en masse de la composition avant séchage et après séchage. Cette amélioration ne nécessite pas la mise en oeuvre d'une étape de broyage mécanique supplémentaire et ne nécessite pas de temps additionnel de dispersion lors de la fabrication de la composition. De préférence, cet ingrédient est ajouté avant l'introduction de la matière pigmentaire et au sein de la même phase que le pigment.

### Exemple 1 : Exemple de mascara

| | | Cas A | Cas B | Cas C Selon l'invention |
|---|---|---|---|---|
| **Phase A** | | | | |
| | Eau | 61.78 | 61.28 | 61.28 |
| | Phenoxyéthanol | 0.50 | 0.50 | 0.50 |
| | Triéthanolamine | 1.50 | 1.50 | 1.50 |
| | Imidazolidinylurée | 0.30 | 0.30 | 0.30 |
| | Hydroxyéthylcellulose | 0.50 | 0.50 | 0.50 |
| | Méthylparaben | 0.20 | 0.20 | 0.20 |
| | PVP | 1.00 | 1.00 | 1.00 |
| | Sorbate de potassium | 0.30 | 0.30 | 0.30 |
| | Mouillant X* | | 0.50 | |
| | Polyester-4 | | | 0.50 |

| **Phase B** | | | | |
|---|---|---|---|---|
| | Oxyde de fer noir | 10.00 | 10.00 | 10.00 |

| **Phase C** | | | | |
|---|---|---|---|---|
| | Acide stéarique | 3.00 | 3.00 | 3.00 |
| | Cire de carnauba | 7.00 | 7.00 | 7.00 |
| | Cire dé paraffine | 7.00 | 7.00 | 7.00 |
| | Collophane | 1.80 | 1.80 | 1.80 |
| | Cétéaryl alcohol | 1.25 | 1.25 | 1.25 |
| | Glycéryl stéarate | 3.50 | 3.50 | 3.50 |

| **Phase D** | | | | |
|---|---|---|---|---|
| | Diméthicone | 0.20 | 0.20 | 0.20 |
| | Propylparaben | 0.15 | 0.15 | 0.15 |
| | BHT | 0.02 | 0.02 | 0.02 |

| | | | | |
|---|---|---|---|---|
| * Mouillant X : Ceraphyl 847 (Nom INCI : OCTYLDODECYL STEAROYL STEARATE) | | | | |

## Revendications

1. Composition de maquillage pour fibres, en particulier de fibres kératiniques, comprenant au moins une phase lipidique, au moins un pigment, au moins un agent filmogène, au moins une phase aqueuse et une quantité efficace d'au moins un agent d'amélioration de l'intensité de la couleur, **caractérisée en ce que** le au moins un agent d'amélioration de l'intensité de la couleur est un copolymère par condensation d'acide adipique et de pentaérythritol comprenant des groupes terminaux d'acide caproïque, d'acide heptanoïque, d'acide caprylique et/ou d'acide caprique ou un mélange de plusieurs de ces copolymères et **en ce que** la composition est soit une émulsion de type huile dans eau comprenant entre 30% et 80% en poids d'eau par rapport à la composition totale, soit une émulsion de type eau dans huile comprenant entre 50% et 95% en poids de composant lipidique par rapport à la composition totale.

2. Composition selon la revendication 1, dans laquelle le au moins un agent d'amélioration de l'intensité de la couleur est le polyester-4.

3. Composition selon la revendication 1 ou 2, dans laquelle le au moins un agent d'amélioration de l'intensité de la couleur représente de 0.01 à 30 % en poids, de préférence de 0.1 à 20 % en poids, en particulier de 0.2 à 10 % en poids de la composition totale.

4. Composition selon l'une quelconque des revendications 1 à 3, qui est une émulsion de type huile dans eau comprenant entre 40% et 70% en poids d'eau par rapport à la composition totale.

5. Composition selon l'une quelconque des revendications 1 à 3, qui est une émulsion de type eau dans huile comprenant entre 60% et 90% en poids de composant lipidique par rapport à la composition totale.

6. Composition selon l'une quelconque des revendications 1 à 5, dans laquelle le au moins un pigment est choisi parmi les pigments inorganiques, notamment les oxydes de fer noirs, bruns, rouges et jaunes, les oxydes de chrome et les hydrates de chrome, le bleu outremer, le bleu ferrique, le dioxyde de titane, le violet de manganèse; les pigments organiques, notamment le noir de carbone, les pigments de type D&C à base de baryum, strontium, calcium et aluminium ou encore le pigment obtenu à partir de la cochenille; et les nacres de mica naturel ou synthétique, éventuellement recouvert de dioxyde de titane, d'oxyde de fer; ainsi que leurs combinaisons.

7. Composition selon l'une quelconque des revendications 1 à 6, dans laquelle le au moins un pigment représente de 0.1 à 15% en poids de la composition totale.

8. Composition selon l'une quelconque des revendications 1 à 7, dans laquelle le au moins un agent filmogène est choisi parmi les polymères d'ester vinylique comme par exemple le polyvinylacétate, ou encore les copolymères d'esters vinyliques; les copolymères de polyvinylpyrrolidone tels que les copolymères vinylpyrrolidone et triacontane, les copolymères vinyl pyrrolidone et éicosène, et les copolymères vinylpyrrolidone et hexadécène; les polymères de cellulose et ses dérivés : sodium hydroxyéthylcellulose, carboxyméthylcellulose, cétyl hydroxyéthylcellulose, hydroxyéthylcellulose; les polyquaterniums; les copolymères éthylène/propylène/styrène ou les copolymères butylène/éthylène/styrène; les polyesters obtenus par polycondensation d'acides dicarboxyliques avec des polyols en particulier des diols; les résines siloxysilicates comme le triméthylsiloxysilicate ou les copolymères de résines de silicone;

9. Composition selon l'une quelconque des revendications 1 à 8, dans laquelle le au moins un agent filmogène est choisi les polymères acryliques sous forme de dispersions aqueuses ou d'émulsions, notamment les copolymères et homopolymères acryliques et les copolymères styrène/acryliques, les polyuréthanes sous forme d'émulsions ou de dispersions aqueuses; ainsi que leurs mélanges.

10. Composition selon l'une quelconque des revendications 1 à 9, dans laquelle le ou les agent(s) filmogène(s) représente(nt) entre 0.5% et 25% en poids, de préférence entre 1 % et 15% en poids de la composition totale.

11. Composition selon l'une quelconque des revendications 1 à 10, comprenant en outre un ou plusieurs autres additifs choisis parmi les charges, les fibres, les agents gélifiants, les agents conservateurs, les antioxydants, les résines d'origine végétale, les agents tensioactifs émulsionnants et des co-émulsionnants, les parfums, les émollients, les vitamines et les filtres solaires.

12. Utilisation d'un copolymère par condensation d'acide adipique et de pentaérythritol comprenant des groupes terminaux d'acide caproïque, d'acide heptanoïque, d'acide caprylique et/ou d'acide caprique ou d'un mélange de plusieurs de ces copolymères dans la préparation d'une composition de maquillage pour fibres, en particulier de fibres kératiniques, comme agent(s) d'amélioration de l'intensité de la couleur.

13. Utilisation selon la revendication 12, dans laquelle la quantité de copolymère(s) par condensation d'acide adipique et de pentaérythritol comprenant des groupes terminaux d'acide caproïque, d'acide heptanoïque, d'acide caprylique et/ou d'acide caprique représente 0.01 à 30 % en poids de la composition totale.

14. Procédé de préparation d'une composition de maquillage pour fibres, en particulier de fibres kératiniques, selon l'une quelconque des revendications 1 à 11, comprenant le mélange d'au moins une phase lipidique, d'au moins un pigment, d'au moins un agent filmogène, d'au moins une phase aqueuse et d'au moins un agent d'amélioration de l'intensité de la couleur, le ou les agent(s) d'amélioration de l'intensité de la couleur étant choisi(s) parmi les copolymères par condensation d'acide adipique et de pentaérythritol comprenant des groupes terminaux d'acide caproïque, d'acide heptanoïque, d'acide caprylique et/ou d'acide caprique ou leurs mélanges, ainsi qu'éventuellement au moins une phase aqueuse, et/ou éventuellement un ou plusieurs autres additifs choisis parmi les charges, les fibres, les agents gélifiants, les agents conservateurs, les antioxydants, les résines d'origine végétale, les agents tensioactifs émulsionnants et des co-émulsionnants, les parfums, les émollients, les vitamines et les filtres solaires.
